# EUROPEAN PATENT SPECIFICATION

(11) **EP 2 532 382 B1**
(45) Date of publication and mention of the grant of the patent: **27.08.2014**
(21) Application number: 12169816.1
(22) Date of filing: 29.05.2012
(51) Int. Cl.: A61M 25/09

(54) **Guidewire**
Führungsdraht
Fil-guide

(30) Priority: 06.06.2011 JP 2011126468
(43) Date of publication of application: 12.12.2012
(73) Proprietor: ASAHI INTECC CO., LTD., Nagoya-shi, Aichi 463-0024 (JP)
(72) Inventor: Maki, Hideaki, Nagoya-shi, Aichi 463-0024 (JP)
(74) Representative: Winter, Brandl, Fürniss, Hübner, Röss, Kaiser, Polte - Partnerschaft mbB

(56) References cited:
- WO-A1-98/42399
- JP-A- 2007 089 901
- US-A- 5 924 998
- US-A1- 2004 122 340

## Description

### BACKGROUND OF THE INVENTION

### 1. Field of the Invention

The invention relates to a medical device. More specifically, the invention relates to a guidewire.

### 2. Description of the Related Art

Hitherto, various guidewires used for guiding, for example, medical devices to target parts by being inserted into body tissues or tubes such as blood vessels, alimentary canals, or ureters, have been proposed. In recent years, many internal treatments using guidewires have been performed on vascularly occluded lesions that cause ischemic diseases. It is known that microchannels exist in the vascularly occluded lesions. Guidewires that are currently being developed are required to have increased insertability and passability with respect to the microchannels in the vascularly occluded lesions.

For example, the guidewire discussed in US 2007/0185415 A1 includes a distal tip portion formed by welding at a distal end of the guidewire. The distal tip portion is formed with decreasing outside diameter towards a distal end or in the form of a chisel, to increase the insertability into vascularly occluded lesions.

The guidewire discussed in JP 2007-089901 A has a protrusion at a distal end of a distal tip portion, to increase the insertability into vascularly occluded lesions.

In the guidewire discussed in US 2007/0185415 A1, since a distal tip portion is formed by welding, it is necessary to melt once the material of a coil and the material of a core in the vicinity of the distal tip portion. Therefore, heat during the welding causes the core and the coil to be in an annealed state, thereby considerably reducing mechanical strength. When the core and the coil are formed of different types of metals, the melting point of the core and the melting point of the coil differ from each other. Therefore, sufficient weld strength cannot be obtained, as a result of which mechanical strength of the distal tip portion is further reduced. Consequently, when the guidewire discussed in US 2007/0185415 A1 is inserted into vascularly occluded lesions, a distal end portion of the guidewire is deformed, as a result of which the guidewire does not have sufficient insertability into the vascularly occluded lesions.

Further, it is difficult to form the distal tip portion with a spherical shape. Therefore, it takes a lot of time to taper the distal tip portion afterwards.

The guidewire discussed in JP 2007-089901 A has increased insertability into vascularly occluded lesions due to the protrusion formed at the distal end of the distal tip portion. However, since the outside diameter increases suddenly from a proximal end of the protrusion towards the distal end of the distal tip portion, the resistance of the guidewire against the vascularly occluded lesions is high. As a result, the guidewire discussed in JP 2007-089901 A does not have sufficient passability with respect to vascularly occluded lesions.

US 5,924,998 A and US 2004/0122340 A1 disclose other examples of guidewires. Further, WO 98/42399 A1 discloses a guidewire in accordance with the preamble of claim 1.

### SUMMARY OF THE INVENTION

Starting from WO 98/42399 A1, it is an object of the present invention to provide a guidewire that has excellent insertability and passability with respect to vascularly occluded lesions, and that makes it possible to achieve high productivity. This object is solved by a guidewire according to claim 1. Preferred embodiments are subject-matter of dependent claims and/or are explained in the following with respects to the drawings.

According to the present invention, there is provided a guidewire including a core shaft, a coil body that covers the core shaft, and a tip portion where a distal end of the coil body and a distal end of the core shaft are joined using metal solder. The tip portion includes an outside diameter decreasing portion where an outside diameter of the tip portion decreases linearly in a direction of a distal end of the tip portion, and a rounded portion, e.g. in form of a hemisphere-shaped portion or a spherical cap-shaped portion, provided at a distal end of the outside diameter decreasing portion. An outside diameter of a proximal end of the rounded portion, i.e. a maximum outside diameter of the rounded portion, is the same as a minimum outside diameter of the distal end of the outside diameter decreasing portion. The coil body has a tapered portion where a coil outside diameter of the coil body decreases linearly in a direction of the distal end of the coil body. Further, a decreasing degree of an outside diameter of the outside diameter decreasing portion is greater than a decreasing degree of a coil outside diameter of the tapered portion.

Since the guidewire according to the present invention is formed using metal solder, it is possible to reduce the influence of heat on the core shaft and the coil to prevent the mechanical strength of the distal end of the guidewire from being reduced, so that, even if the guidewire is inserted into vascularly occluded lesions, it is possible to prevent deformation of the distal end portion of the guidewire, thereby making it is possible to increase the insertability of the guidewire into the vascularly occluded lesions. In addition, since metal solder is used, it possible to easily form the tip portion at the distal end of the guidewire, and to achieve excellent productivity. Further, since the distal end of the distal tip portion is provided with a rounded portion whose outside diameter decreases towards and is smallest at a distal end of the tip portion, it is possible to move the guidewire to microchannels in the vascularly occluded lesions. Still further, since, when extending from the distal end of the outside-diameter decreasing portion to the proximal end of the rounded portion, the outside diameter of the tip portion is the same or remains constant, i.e. a minimum outside diameter at the distal end of the outside diameter decreasing portion is the same as a maximum outside diameter at the proximal end of the rounded portion, it is possible to prevent the guidewire from being caught in the vascularly occluded lesions, and to increase the insertability and the passability of the guidewire with respect to the vascularly occluded lesions.

Further, the decreasing degree of the outside diameter of the outside diameter decreasing portion is greater than the decreasing degree of the outside diameter of the tapered portion. Therefore, at a boundary portion between a proximal end of the outside diameter decreasing portion and a distal end of the tapered portion, it is possible to reduce resistance in a vascularly occluded lesion when the guidewire passes through the vascularly occluded lesion. This makes it possible to increase passability of the guidewire 11 with respect to the vascularly occluded lesion.

### BRIEF DESCRIPTION OF THE DRAWINGS

Figs. 1A to 1E illustrate structures of a guidewire according to an example embodiment not covered by the claims, with Fig. 1A illustrating the entire guidewire, Fig. 1B being an enlarged view of a tip portion illustrated in Fig. 1A, Fig. 1C illustrating the tip portion as viewed from a distal end of the guidewire towards a proximal end of the guidewire, and Figs. 1D and 1E illustrating modifications of the example embodiment and being enlarged views of the tip portion.
Fig. 2 illustrates an entire guidewire according to a first embodiment of the present invention.
Fig. 3 illustrates a structure of a guidewire according to a second embodiment of the present invention, with Fig. 3 illustrating the entire guidewire.

### DESCRIPTION OF THE PREFERRED EMBODIMENTS

Starting with the description of an example embodiment which is not covered by the claims, guidewires according to the present invention will hereunder be described on the basis of preferred embodiments shown in the drawings.

### Example Embodiment

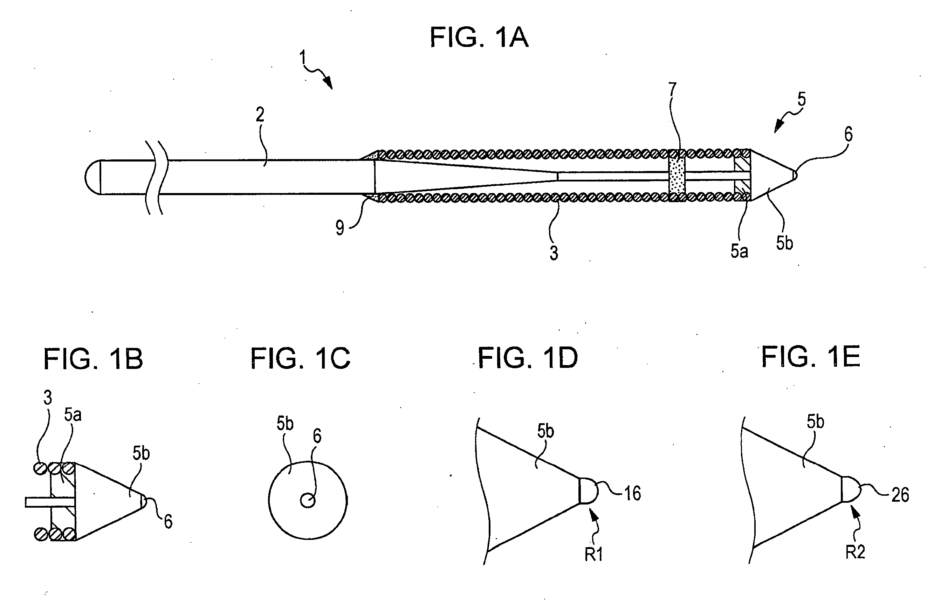

Figs. 1A to 1E illustrate a guidewire 1 according to an example embodiment which is not covered by the claims.

In Figs. 1A to 1E, for convenience of explanation, the left side corresponds to a proximal end and the right side corresponds to a distal end.

In Fig. 1A, for facilitating understanding, a lengthwise direction of the guidewire 1 is reduced. In addition, since Fig. 1A schematically illustrates the entire guidewire 1, the dimensions of the entire guidewire 1 differ from the actual dimensions thereof.

In Fig. 1A, the guidewire 1 includes a core shaft 2, a coil body 3 that covers a distal end of the core shaft 2, and a tip portion 5 where a distal end of the coil body 3 and the distal end of the core shaft 2 are joined. In the direction of a proximal end of the tip portion 5, the coil body 3 and the core shaft 2 are joined by an intermediate joining portion 7, and a proximal end of the coil body 3 and the core shaft 2 are joined by a proximal-end joining portion 9.

The tip portion 5 includes a proximal-end tip portion 5a, an outside diameter decreasing portion 5b, and a rounded portion in form of a spherical cap-shaped portion 6. The distal end of the coil body 3 and the distal end of the core shaft 2 are joined at the proximal-end tip portion 5a. The outside diameter of the outside diameter decreasing portion 5b decreases linearly in the direction of the distal end. The spherical cap-shaped portion 6 is provided at a distal end of the outside diameter decreasing portion 5b.

In Fig. 1B, at a transition or boundary portion between the distal end of the outside diameter decreasing portion 5b and a proximal end of the spherical cap-shaped portion 6 the tip portion 5 has a constant outside diameter. The aforementioned transition or boundary portion can be an interface between the outside diameter decreasing portion 5b and the spherical cap-shaped portion 6 or a longitudinally extended portion formed by the outside diameter decreasing portion 5b and/or the spherical cap-shaped portion 6, i.e., the boundary portion may have an extension in a longitudinal direction of the guidewire 1. Accordingly, the guidewire 1 is formed so that its state smoothly changes from a state in which the outside diameter of the outside diameter decreasing portion 5b gradually decreases linearly, and, then, to a state in which the outside diameter of the spherical cap-shaped portion 6 decreases so as to form a curve with respect to the longitudinal cross-sectional view of the guidewire 1 (cf. Fig. 1B). In other words, the spherical cap-shaped portion 6 has a curved or rounded outer contour in a direction towards a distal end of the guidewire 1.

When the guidewire 1 shown in Fig. 1B is viewed from the distal end of the guidewire 1 towards the proximal end of the guidewire 1, the outside diameter decreasing portion 5b and the spherical cap-shaped portion 6 have circular shapes that are not distorted in a particular way in a particular direction as shown in Fig. 1C.

Accordingly, the tip portion 5 of the guidewire 1 has the outside diameter decreasing portion 5b whose outside diameter decreases linearly towards the distal end, and the spherical cap-shaped portion 6 provided at the distal end of the outside diameter decreasing portion 5b. In addition, the outside diameter at the boundary portion between the distal end of the outside diameter decreasing portion 5b and the proximal end of the spherical cap-shaped portion 6 is constant. Therefore, it becomes easier to insert the hemisphere-shaped portion 6 into an entrance of a microchannel in a vascularly occluded lesion, and the guidewire 1 can be easily passed through the interior of the vascularly occluded lesion without receiving a high resistance even when the guidewire 1 passes through the interior of the vascularly occluded lesion. The form of the guidewire 1 makes it possible to increase the insertability and passability of the guidewire 1 with respect to the interior of the vascularly occluded lesion.

Since the outside diameter of the outside diameter decreasing portion 5b decreases linearly towards the distal end, it is possible to further reduce the resistance applied to the guidewire 1 when the guidewire 1 is inserted into the vascularly occluded lesion. Therefore, it is possible to further increase the insertability of the guidewire 1 into the vascularly occluded lesion.

The outside diameter decreasing portion 5b and the spherical cap-shaped portion 6 have circular shapes that are not distorted in a particular way in a particular direction. Therefore, when the spherical cap-shaped portion 6 is inserted into a microchannel in a vascularly occluded lesion, it is possible to prevent the spherical cap-shaped portion 6 from being caught by an entrance of the microchannel. Consequently, it is possible to insert the guidewire 1 into the microchannel in the vascularly occluded lesion, and the guidewire 1 is not often subjected to resistance in the interior of the vascularly occluded lesion even when the guidewire 1 is pushed into and passed through the interior of the vascularly occluded lesion or passed through the interior of the vascularly occluded lesion while rotating the guidewire 1. Thus, by forming the outside diameter decreasing portion 5b and the spherical cap-shaped portion 6 with such circular shapes, it is possible to further increase the insertability and the passability of the guidewire 1 with respect to the interior of the vascularly occluded lesion.

Fig. 1D illustrates a modification of the example embodiment. Comparing a rounded portion in form of the hemisphere-shaped portion 16 as illustrated in Fig. 1D with the rounded portion in form of the spherical cap-shaped portion 6 illustrated in Fig. 1A, the hemisphere-shaped portion 16 has a completely hemispherical shape (that is, has a shape that is half a sphere). By forming the hemisphere-shaped portion 16 with a completely hemispherical shape, the hemisphere-shaped portion 16 protrudes in the direction of the distal end of the guidewire 1. At a boundary portion between the distal end of the outside diameter decreasing portion 5b and the proximal end of the hemisphere-shaped portion 16, the distal end of the outside diameter decreasing portion 5b and a proximal end of the hemisphere-shaped portion 16 are connected to each other so as to form a curve R1.

Accordingly, when the hemisphere-shaped portion 16 having a completely hemispherical shape is provided at the guidewire 1, the hemisphere-shaped portion 16 protrudes in the direction of the distal end of the guidewire 1. As a result, it becomes easier to move the guidewire 1 to a microchannel in a vascularly occluded lesion, thereby making it possible to further increase the insertability of the guidewire 1 into the vascularly occluded lesion.

Further, since, at the boundary portion between the distal end of the outside diameter decreasing portion 5b and the proximal end of the hemisphere-shaped portion 16, the distal end of the outside diameter decreasing portion 5b and the proximal end of the hemisphere-shaped portion 16 are connected to each other so as to form the curve R1, it is possible to further reduce resistance applied to the boundary portion when the boundary portion passes through the vascularly occluded lesion. Consequently, it is possible to further increase passability of the guidewire 1 with respect to the vascularly occluded lesion.

Fig. 1E also shows a modification of the example embodiment. A rounded portion in form of a hemisphere-shaped portion 26 illustrated in Fig. 1E has a spindle-like hemispherical shape. Accordingly, comparing the hemisphere-shaped portion 26 with the spherical cap-shaped portion 6 shown in Fig. 1A, the hemisphere-shaped portion 26 protrudes by a greater amount in the direction of the distal end of the guidewire 1. In particular, the near proximal end of the hemisphere-shaped portion 26 having a spindle-like hemispherical shape, has a short constant-diameter portion joined to the distal end of the outside diameter decreasing portion 5a. At a boundary portion between the distal end of the outside diameter decreasing portion 5b and a proximal end of the hemisphere-shaped portion 26, the distal end of the outside diameter decreasing portion 5b and the proximal end of the hemisphere-shaped portion 26 are connected to each other so as to form a curve R2.

Accordingly, since the spindle-like hemisphere-shaped portion 26 protrudes by a greater amount in the direction of the distal end of the guidewire 1, it becomes easier to move the guidewire 1 to a microchannel of a vascularly occluded lesion. Therefore, it is possible to further increase the insertability of the guidewire 1 into the vascularly occluded lesion. In the addition, at the boundary portion between the distal end of the outside diameter decreasing portion 5b and the proximal end of the hemisphere-shaped portion 26, the distal end of the outside diameter decreasing portion 5b and the proximal end of the hemisphere-shaped portion 26 are connected to each other so as to form the curve R2. This makes it possible for the boundary portion between the distal end of the outside diameter decreasing portion 5b and the proximal end of the hemisphere-shaped portion 26 to further reduce the resistance applied to the boundary portion when the boundary portion passes through the interior of the vascularly occluded lesion. Therefore, it is possible to further increase the passability of the guidewire 1 with respect to the vascularly occluded lesion.

Next, the materials of the structural parts in the embodiment will be described. Although the usable materials of the core shaft 2 are not particularly limited, materials such as stainless steel (such as SUS304), superelastic alloys (such as Ni-Ti alloys), and piano wires may be used.

Examples of the materials of the tip portion 5, the intermediate joining portion 7, and the proximal-end joining portion 9 for joining the core shaft 2 and the coil body 3 include metal solders, such as Sn-Pb solders, Pb-Ag solders, Sn-Ag solders, and Au-Sn solders. In order to reduce a reduction in mechanical strength caused by the influence of heat on the core shaft 2 and on the coil body 3, it is desirable to use metal solders having melting points that are lower than those of the materials of the coil body 3 and the materials of the core shaft 2 such as those mentioned above. Further, in order to reliably prevent a reduction in mechanical strength caused by the influence of heat on the core shaft 2 and on the coil body 3, it is desirable to use metal solders having melting points that are equal to or less than 500 degrees.

In particular, it is desirable that, for the tip portion 5, metal solders such as Au-Sn solders whose principal component is gold be used. Metal solders, such as Au-Sn solders, are known to have high rigidity. When the tip portion 5 is formed of an Au-Sn alloy, the tip portion 5 can have the proper rigidity, thereby increasing the insertability of the guidewire 1 into a vascularly occluded lesion. The melting point of Au-Sn alloy is equal to or less than 400 degrees, so that it is a metal solder that makes it possible to prevent a reduction in mechanical strength caused by the influence of heat on the core shaft 2 and on the coil body 3.

Since metal solders, such as Au-Sn solders, are highly impermeable to radiation, it can increase visibility for a radiation transparent image at the tip portion 5 of the guidewire 1. As a result, it is possible to operate the guidewire 1 while definitely knowing the position of the guidewire 1 in a vascularly occluded lesion. This is advantageous when the guidewire 1 is being passed through the interior of the vascularly occluded lesion.

When assembling the core shaft 2 and the coil body 3 using metal solder, it is desirable to previously apply flux to a portion where the core shaft 2 and the coil body 3 are to be joined. This results in good wettability of the metal solder with respect to the core shaft 2 and the coil body 3, and to increase bonding strength.

By forming the tip portion 5 of the guidewire 1 using metal solder in this way, it is possible to suppress a reduction in mechanical strength caused the influence of heat on the coil body 3 and the core shaft 2 of the guidewire 1. This makes it possible to ensure mechanical strength of a distal end portion of the guidewire 1. Consequently, it is possible to increase the insertability of the guidewire 1 into a vascularly occluded lesion. In addition, as described below, metal solders can be used to easily form the tip portion 5 by using, for example, a soldering iron, so that it is possible to achieve excellent productivity when forming the tip portion 5 of the guidewire 1.

Usable materials of the coil body 3 include wires that are impermeable to radiation and wires that are permeable to radiation.

Usable materials of the wires that are impermeable to radiation are not particularly limited, and include gold, platinum, tungsten, and alloys of these elements (such as platinum-nickel alloys).

Usable materials of the wires that are permeable to radiation are not particularly limited, and include stainless steel (SUS304, SUS316, etc.), superelastic alloys (such as Ni-Ti alloys), and piano wires.

The guidewire 1 according to the example embodiment may be manufactured by the following method.

First, an outer periphery of one end of a metal wire is ground by a centerless grinding machine, to manufacture a core shaft 2 whose distal end portion has a smaller outside diameter.

Next, coil wires are wound around a cored bar for a coil, and the coil wires are subjected to heat treatment while they are wound around the cored bar, after which the cored bar is removed, thereby manufacturing a coil body 3.

Then, a distal end of the core shaft 2 is inserted from a proximal end of the coil body 3, and the proximal end of the coil body 3 and the core shaft 2 are joined together with metal solder using, for example, a soldering iron, to form a proximal-end joining portion 9.

Next, using, for example, the soldering iron, a distal end of the coil body 3 and the distal end of the core shaft 2 are joined using metal solder. At this time, a proximal-end tip portion 5a and a previous form portion are formed. The proximal-end tip portion 5a is joined to the distal end of the core shaft 2 when metal solder enters the interior of the coil body 3. The previous form portion has an overall hemispherical shape and is formed at a distal end side of the proximal-end tip portion 5a.

Next, in the direction of a proximal end of the proximal-end tip portion 5a, the core shaft 2 and the coil body 3 are joined using metal solder, to form an intermediate joining portion 7.

Lastly, using a device, such as Leutor (grinding tool), the previous form portion is ground, to form an outside diameter decreasing portion 5b and a rounded (e.g., spherical cap-shaped or hemisphere-shaped) portion 6.

From the viewpoint of strength, it is desirable to integrally form the outside diameter decreasing portion 5b and the rounded (e.g., spherical cap-shaped or hemisphere-shaped) portion 6 from the previous form portion, as above described. However, the outside diameter decreasing portion 5b and rounded portion 6 may be formed separately or successively. That is, after forming the outside diameter decreasing portion 5b a previous form of the rounded portion 6 could be formed using, for example, metal solder, which previous form of the rounded portion 6 is then ground by using a device, such as Leutor, to finish the rounded portion 6. After forming the previous form portion of the rounded portion 6 using, for example, metal solder after forming the outside diameter decreasing portion 5b, it is possible to grind the previous form portion again, and adjust its shape using, for example, Leutor. When, for example, metal solder is further used after forming the outside diameter decreasing portion 5b, it is possible to use a different type of metal solder. However, considering the welding temperature and the joining strength with the outside diameter decreasing portion 5b, it is desirable to use the same type of metal solder.

The manufacturing method of the guidewire 1 is not limited to this manufacturing method, so that the tip portion 5 may be manufactured by other publicly known methods. For example, it is possible to, by using a die whose hollow has the shape of the outside diameter decreasing portion 5b and the shape of the rounded portion 6, set the distal end of the core shaft 2 and the distal end of the coil body 3, and pour molten metal solder into the die, to form the tip portion 5.

Although, in the example embodiment, the distal end of the core shaft 2 is disposed in the proximal-end tip portion 5a, the present invention is not limited thereto. It is possible to dispose the distal end of the core shaft 2 in the outside diameter decreasing portion 5b or the rounded portion 6. When the distal end of the core shaft 2 is disposed in the outside diameter decreasing portion 5b or the rounded portion 6, it is possible to increase the push-in characteristic of the guidewire 1. Therefore, it is possible to increase the insertability and passability of the guidewire 1 with respect to a vascularly occluded lesion.

### First Embodiment

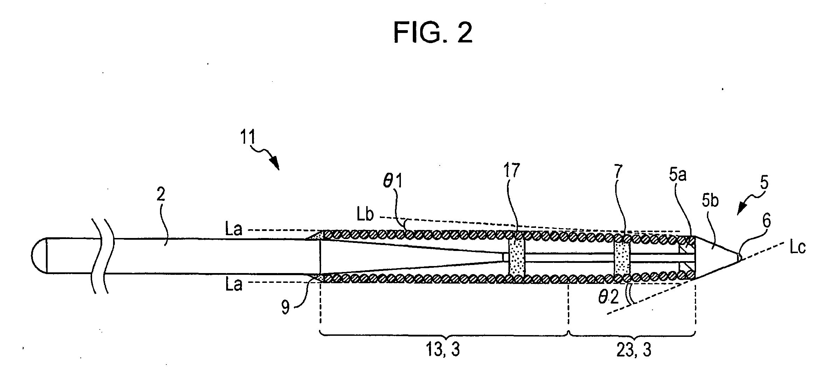

Next, a guidewire 11 according to a first embodiment will be described with reference to Fig. 2 by focusing on the differences between it and the guidewire 1 according to the example embodiment. Portions that correspond to those in the example embodiment will be given the same reference numerals in Fig. 2.

In Fig. 2, for facilitating understanding, a lengthwise direction of the guidewire 11 is reduced, and the entire guidewire 11 is schematically illustrated, so that the dimensions of the entire guidewire 11 differ from the actual dimensions thereof.

In Fig. 2, the guidewire 11 has the same structural features as the guidewire 1 according to the example embodiment except that the guidewire 11 includes a constant outside diameter portion 13, a tapered portion 23, and an intermediate joining portion 7. The constant outside diameter portion 13 is where a proximal end side of a coil body 3 has a constant coil outside diameter. The tapered portion 23 is where a distal end side of the coil body 3 has a coil outside diameter that decreases in a direction of a distal end. The second intermediate joining portion 17 is where a core shaft 2 and the coil body 3 are joined in a direction of a proximal end of the second intermediate joining portion 17.

Here, in the coil body 3 shown in Fig. 2, a tangent line that contacts a coil outer periphery at the constant outside diameter portion 13 is defined as a straight line La, a tangent line that contacts the coil outer periphery at the tapered portion 23 is defined as a straight line Lb, and, in a tip portion 5 shown in Fig. 2, a tangent line that contacts an outer periphery of an outside diameter decreasing portion 5b is defined as a straight line Lc. In this case, an angle formed between the straight lines La and Lb is angle θ1. The angle θ1 corresponds to a taper angle of the tapered portion 23 with respect to a center axis of the guidewire 11 in a long-axis direction of the guidewire 11. The angle formed between the straight lines La and Lc is angle θ2. The angle θ2 is an angle by which the outside diameter of the outside diameter decreasing portion 5b decreases with respect to the center axis of the guidewire 11 in the long-axis direction of the guidewire 11.

In the first embodiment, the angle θ2 by which the outside diameter of the outside diameter decreasing portion 5b decreases is set so as to be larger than the angle θ1 of the tapered portion 23 so that the decreasing degree of the outside diameter of the outside diameter decreasing portion 5b is greater than the decreasing degree of the outside diameter of the tapered portion 23.

Accordingly, in the guidewire 11 according to the first embodiment, the decreasing degree of the outside diameter of the outside diameter decreasing portion 5b is greater than the decreasing degree of the outside diameter of the tapered portion 23. Therefore, at a boundary portion between a proximal end of the outside diameter decreasing portion 5b and a distal end of the tapered portion 23, it is possible to reduce resistance in a vascularly occluded lesion when the guidewire 11 passes through the vascularly occluded lesion. This makes it possible to increase passability of the guidewire 11 with respect to the vascularly occluded lesion.

The coil body 23 including the constant outside diameter portion 13 and the tapered portion 23 can be manufactured by providing, at a cored bar for a coil, a portion whose outside diameter is constant and a portion whose outside diameter decreases, and by using the cored bar when forming the coil body 3.

In the guidewire 11 according to the first embodiment, the decreasing degree of the outside diameter of the outside diameter decreasing portion may be any degree as long as it is greater than the decreasing degree of the outside diameter of the tapered portion 23. For example, the outside diameter of the tapered portion 23 of the coil body 3 and the outside diameter of the outside diameter decreasing portion 5b may decrease so as to form an asymptotic curve.

The second intermediate joining portion 17 may be formed of the same material as the intermediate joining portion 7. The guidewire 11 according to the first embodiment includes the second intermediate joining portion 17 where the constant outside diameter portion 13 of the coil body 3 and the core shaft 2 are joined, and the intermediate joining portion 7 where the tapered portion 23 and the core shaft 2 are joined. This makes it possible to join each portion of the coil body 3 and the core shaft 2. Therefore, the insertability and passability of the guidewire 11 with respect to a vascularly occluded lesion can be increased.

### Second Embodiment

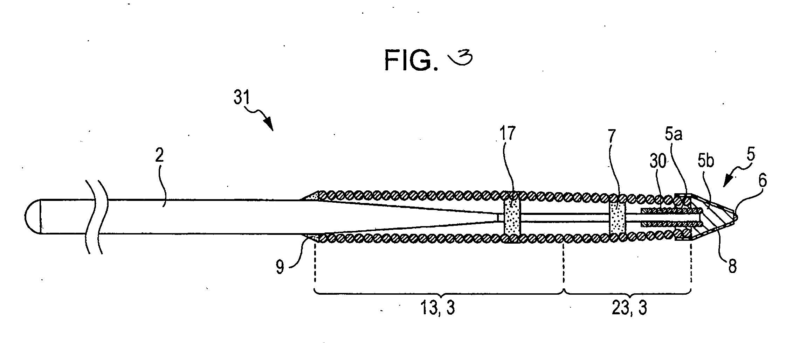

Next, with reference to Fig. 3, a guidewire 31 according to a second embodiment will be described by focusing on the differences between it and the guidewire 1 according to the example embodiment. Portions that correspond to those in the example embodiment will be given the same reference numerals in Fig. 3.

In Fig. 3, for facilitating understanding, a lengthwise direction of the guidewire 31 is reduced, and the entire guidewire 31 is schematically illustrated, so that the dimensions of the entire guidewire 31 differ from the actual dimensions thereof.

In Fig. 3, the guidewire 31 has the same structural features as the guidewire 11 according to the second embodiment except that an inner coil body 30 that covers a distal end portion of a core shaft 2 is provided at an inner side of a distal end portion of a coil body 3, that is, at an inner side of a tapered portion 23, and that a lubricant coating 8 is applied to a portion other than a rounded, e.g., spherical cap-shaped or hemisphere-shaped, portion 6 of a tip portion 5.

The inner coil body 30 which is a multi-thread coil body is a formed by twisting a plurality of coil wires. A distal end of the inner coil body 30 is joined along with a distal end of the coil body 3 and a distal end of the core shaft 2 by the tip portion 5. A proximal end of the inner coil body 30 is joined to the core shaft 2 at a position that is closer to a proximal end side than the tip portion 5 and that is closer to a distal end side than an intermediate joining portion 7. The distal end of the inner coil body 30 is disposed closer to the distal end side than the distal end of the coil body 3, and inside an outside diameter decreasing portion 5b of the tip portion 5.

Accordingly, in the guidewire 31 according to the second embodiment, the proximal end of the inner coil body 30 formed of a multi-thread coil body is joined to the core shaft 2. While the distal end of the inner coil body 30 is disposed closer to the distal end side than the distal end of the coil body 3, and is positioned in the outside diameter decreasing portion 5b of the tip portion 5, the inner coil body 30 is joined to the distal end of the core shaft 2 and the distal end of the coil body 3 through the tip portion 5. This makes it possible to more easily transmit, for example, a push-in force at a proximal end side of the guidewire 31 to the tip portion 5, so that the insertability of the guidewire 31 into a vascularly occluded lesion is considerably increased.

The inner coil body 30 formed of a multi-thread coil body may be formed of a material that is the same as the material of the coil body 3. However, from the viewpoint of transmitting, for example, a push-in force at the proximal end side of the guidewire 31, it is desirable to use, for example, stainless steel wires having excellent mechanical strength as coil wires of the inner coil body 30.

The method of forming the multi-thread coil body 30 may be the same as the method of forming the coil body 3 except for the winding of a plurality of coil wires around a cored bar.

The present invention is not limited to the above-described embodiments. Various modifications can be made by those skilled in the art within the technical idea of the present invention as defined by the claims.

For example, in order to increase the insertability and passability of the guidewire 31 with respect to a vascularly occluded lesion, it is desirable to form the inner coil body 30 of the guidewire 31 according to the second embodiment shown in Fig. 3 using a multi-thread coil body. However, when the distal end portion of the guidewire 31 is required to have flexibility, a single coil body formed of one coil wire may be used.

## Claims

1. A guidewire (11, 31) comprising:
a core shaft (2);
a coil body (3) that covers the core shaft (2); and
a tip portion (5) where a distal end of the coil body (3) and a distal end of the core shaft (2) are joined to each other, wherein
the distal end of the coil body (3) and the distal end of the core shaft (2) are joined to each other using metal solder,
the tip portion (5) includes an outside diameter decreasing portion (5b) where an outside diameter of the tip portion (5) decreases linearly in a direction of a distal end of the tip portion (5), and a rounded portion (6, 16, 26) provided at a distal end of the outside diameter decreasing portion (5b),
a maximum outside diameter of a proximal end of the rounded portion (6, 16, 26) is the same as a minimum outside diameter of the distal end of the outside diameter decreasing portion (5b), and
the coil body (3) has a tapered portion (23) where a coil outside diameter of the coil body (3) decreases in a direction of the distal end of the coil body (3), **characterized in that**
a coil outside diameter of the tapered portion (23) of the coil body (3) decreases linearly in a direction of the distal end of the coil body (3), and
a decreasing degree of an outside diameter of the outside diameter decreasing portion (5b) is greater than a decreasing degree of a coil outside diameter of the tapered portion (23).

2. The guidewire according to Claim 1, wherein, at a boundary portion between the outside diameter decreasing portion (5b) and the rounded portion (6, 16, 26), the outside diameter decreasing portion (5b) and the rounded portion (6, 16, 26) are connected to each other so as to form a curved outer contour (R1, R2).

3. The guidewire according to any one of Claims 1 and 2, wherein the tip portion (5) is coated with a lubricant coating (8).

4. The guidewire according to any one of Claims 1 to 3, further comprising a multi-thread coil body (30) at an inner side of the coil body (3), the multi-thread coil body (30) covering the core shaft (2),
wherein a proximal end of the multi-thread coil body (30) is joined to the core shaft (2) in the tapered portion (23), and
wherein a distal end of the multi-thread coil body (30) is disposed closer to a distal end side than the distal end of the coil body (3), and is joined to the core shaft (2) in the outside diameter decreasing portion (5b) through the tip portion (5).

## Patentansprüche

1. Führungsdraht (11, 31) mit:
einem Kern (2);
einer den Kern (2) umhüllenden Wicklung (3); und
einer Spitze (5), an der das distale Ende der Wicklung (3) und das distale Ende des Kerns (2) miteinander verbunden sind, wobei
das distale Ende der Wicklung (3) und das distale Ende des Kerns (2) mittels eines Metalllots miteinander verbunden sind,
die Spitze (5) einen Außendurchmesserverjüngungsabschnitt (5b), in dem der Außendurchmesser der Spitze (5) in Richtung eines distalen Endes der Spitze (5) linear abnimmt, und einen am distalen Ende des Außendurchmesserverjüngungsabschnitts (5b)vorgesehenen Rundungsabschnitt (6, 16, 26) aufinreist,
ein maximaler Außendurchmesser des proximalen Endes des Rundungsabschnitts (6, 16, 26) gleich einem minimalen Außendurchmesser des distalen Endes des Außendurchmesserverjüngungsabschnitts (5b) ist, und
die Wicklung (3) einen verjüngten Abschnitt (23) aufweist, in dem der Auβendurchmesser der Wicklung (3) in Richtung des distalen Endes der Wicklung (3) abnimmt, **dadurch gekennzeichnet, dass**
der Außendurchmesser des verjüngten Abschnitts (23) der Wicklung (3) in Richtung des distalen Endes der Wicklung (3) linear abnimmt, und
der Außendurchmesser des Außendurchmesserverjüngungsabschnitts (5b) stärker abnimmt als der Außendurchmesser des verjüngten Abschnitts (23).

2. Führungsdraht nach Anspruch 1, wobei an der Schnittstelle zwischen dem Außendurchmesserverjüngungsabschnitt (5b) und dem Rundungsabschnitt (6, 16, 26) derAußendurchmesserverjüngungsabschnitt (5b) und der Rundungsabschnitt (6, 16, 26) in der Weise miteinander verbunden sind, dass sie eine gekrümmte Außenkontur (R1, R2) bilden.

3. Führungsdraht nach Anspruch 1 oder 2, wobei die Spitze (5) mit einem Schmiermittelüberzug (8) überzogen ist.

4. Führungsdraht nach einem der Ansprüche 1 bis 3, des Weiteren mit einer den Kern (2) umhüllenden Mehrstrangwicklung (30) innerhalb der Wicklung (3),
wobei das proximale Ende der Mehrstrangwicklung (30) in dem verjüngten Abschnitt (23) mit dem Kern (2) verbunden ist, und
wobei das distale Ende der Mehrstrangwicklung (30) distaler liegt als das distale Ende der Wicklung (3) und durch die Spitze (5) in dem Außendurchmesserverjüngungsabschnitt (5b) mit dem Kern (2) verbunden ist.

## Revendications

1. Fil-guide (11, 31) comprenant :
une broche d'enroulage (2) ;
un corps de bobine (3) qui recouvre la broche d'enroulage (2) ; et
une partie de pointe (5) où une extrémité distale du corps de bobine (3) et une extrémité distale de la broche d'enroulage (2) sont assemblées entre elles, dans lequel :
l'extrémité distale du corps de bobine (3) et l'extrémité distale de la broche d'enroulage (2) sont assemblées entre elles à l'aide d'une soudure métallique,
la partie de pointe (5) comprend une partie décroissante de diamètre extérieur (5b) où un diamètre extérieur de la partie de pointe (5) diminue de manière linéaire dans une direction d'une extrémité distale de la partie de pointe (5), et une partie arrondie (6, 16, 26) prévue au niveau d'une extrémité distale de la partie décroissante de diamètre extérieur (5b),
un diamètre extérieur maximum d'une extrémité proximale de la partie arrondie (6, 16, 26) est le même qu'un diamètre extérieur minimum de l'extrémité distale de la partie décroissante de diamètre extérieur (5b), et
le corps de bobine (3) a une partie progressivement rétrécie (23) où un diamètre extérieur de bobine du corps de bobine (3) diminue dans une direction de l'extrémité distale du corps de bobine (3),
**caractérisé en ce que** :
un diamètre extérieur de bobine de la partie progressivement rétrécie (23) du corps de bobine (3) diminue de manière linéaire dans une direction de l'extrémité distale du corps de bobine (3), et
un degré décroissant d'un diamètre extérieur de la partie décroissante de diamètre extérieur (5b) est supérieur à un degré décroissant d'un diamètre extérieur de bobine de la partie progressivement rétrécie (23).

2. Fil-guide selon la revendication 1, dans lequel, au niveau d'une partie de limite entre la partie décroissante de diamètre extérieur (5b) et la partie arrondie (6, 16, 26) la partie décroissante de diamètre extérieur (5b) et la partie arrondie (6, 16, 26) sont raccordées entre elles afin de former un contour externe incurvé (R1, R2).

3. Fil-guide selon l'une quelconque des revendications 1 et 2, dans lequel la partie de pointe (5) est recouverte avec un revêtement lubrifiant (8).

4. Fil-guide selon l'une quelconque des revendications 1 à 3, comprenant en outre un corps de bobine multifils (30) au niveau d'un côté interne du corps de bobine (3), le corps de bobine multifils (30) recouvrant la broche d'enroulage (2),
dans lequel une extrémité proximale du corps de bobine multifils (30) est assemblée à la broche d'enroulage (2) dans une partie progressivement rétrécie (23), et
dans lequel une extrémité distale du corps de bobine multifils (30) est disposée plus à proximité d'un côté d'extrémité distale que l'extrémité distale du corps de bobine (3) et est assemblée à la broche d'enroulage (2) dans la partie décroissante de diamètre extérieur (5b) par la partie de pointe (5).
